(19) Europäisches Patentamt
European Patent Office
Office européen des brevets

(11) **EP 3 555 686 B1**

(12) **EUROPÄISCHE PATENTSCHRIFT**

(45) Veröffentlichungstag und Bekanntmachung des Hinweises auf die Patenterteilung:
**12.10.2022 Patentblatt 2022/41**

(21) Anmeldenummer: **17808916.5**

(22) Anmeldetag: **05.12.2017**

(51) Internationale Patentklassifikation (IPC):
*G02B 23/24* (2006.01)   *G02B 17/04* (2006.01)
*G02B 27/00* (2006.01)

(52) Gemeinsame Patentklassifikation (CPC):
**G02B 23/2423; G02B 17/045; G02B 27/0018**

(86) Internationale Anmeldenummer:
**PCT/EP2017/081455**

(87) Internationale Veröffentlichungsnummer:
**WO 2018/108618 (21.06.2018 Gazette 2018/25)**

(54) **OPTISCHES SYSTEM FÜR EIN SEITWÄRTS BLICKENDES ENDOSKOP SOWIE SEITWÄRTS BLICKENDES ENDOSKOP**

OPTICAL SYSTEM FOR A SIDE-VIEWING ENDOSCOPE, AND SIDE-VIEWING ENDOSCOPE

SYSTÈME OPTIQUE POUR UN ENDOSCOPE À VISÉE LATÉRALE AINSI QU'ENDOSCOPE À VISÉE LATÉRALE

(84) Benannte Vertragsstaaten:
**AL AT BE BG CH CY CZ DE DK EE ES FI FR GB GR HR HU IE IS IT LI LT LU LV MC MK MT NL NO PL PT RO RS SE SI SK SM TR**

(30) Priorität: **15.12.2016 DE 102016225097**

(43) Veröffentlichungstag der Anmeldung:
**23.10.2019 Patentblatt 2019/43**

(73) Patentinhaber: **OLYMPUS Winter & Ibe GmbH 22045 Hamburg (DE)**

(72) Erfinder:
• ZHAO, Jianxin
22399 Hamburg (DE)
• THÜMEN, Alrun
22043 Hamburg (DE)

(74) Vertreter: **Seemann & Partner Patentanwälte mbB Raboisen 6 20095 Hamburg (DE)**

(56) Entgegenhaltungen:
WO-A1-01/01186        DE-A1- 2 458 306
DE-A1-102016 214 025    US-A- 4 655 557

**Beschreibung**

Die Erfindung betrifft ein optisches System für ein seitwärts blickendes Endoskop, mit einem zentralen Strahlengang, der distal einen seitlichen Blickwinkel $\theta$ in Bezug auf eine Längsachse eines Endoskopschafts des Endoskops aufweist, sowie ein entsprechendes seitwärts blickendes Endoskop.

Die optischen Elemente eines optischen Systems, in der Regel eine oder mehrere Linsen, bilden aus einem Sichtfeld einfallende Lichtbündel auf eine lichtempfindliche Fläche eines Bildsensors ab. Diese Lichtbündel verlaufen innerhalb eines Strahlengangs, der durch die optischen Baugruppen des optischen Systems, genauer durch deren optische Elemente, festgelegt ist.

Das Sichtfeld des optischen Systems bezeichnet einen Bereich oder einen Intervall von Bildwinkeln, innerhalb dessen Ereignisse oder Veränderungen im Objektraum mit dem optischen System wahrgenommen werden können. Aus dem Sichtfeld einfallende Lichtbündel werden auf die lichtempfindliche Fläche des Bildsensors abgebildet. Bei einem rechteckigen Bildsensor ist das Sichtfeld durch einen horizontalen Bildwinkel und durch einen vertikalen Bildwinkel definiert. Der horizontale und vertikale Bildwinkel sind durch die Ränder des Aufnahmeformats begrenzt, welches wiederum von der Größe und Form des Bildsensors abhängt. Bei einem rechteckigen Bildsensor ist der vertikale Bildwinkel typischerweise kleiner als der horizontale Bildwinkel (Querformat). Der horizontale Bildwinkel und der vertikale Bildwinkel sind also die maximal möglichen Einfallswinkel, unter denen Lichtstrahlen in das optische System einfallen können und gerade noch auf der lichtempfindlichen Fläche des Bildsensors abgebildet werden.

Fallen Lichtbündel unter größeren Winkeln in das optische System ein, kommt es zu Reflektionen an den optischen Elementen. Ebenso verursachen diese Lichtbündel diffuse Streuung oder Reflexion an einem Tubus oder Linsenrohr, in dem die optischen Elemente des optischen Systems aufgenommen sind. Diese Reflektionen, die vielfach auch als "Flare" oder "Lens Flare" bezeichnet werden, beeinflussen die Bildqualität des optischen Systems nachteilig. Traditionell wird der Einfall solcher Lichtbündel durch mechanische Masken oder Blenden innerhalb des optischen Systems verringert. Masken oder Blenden führen jedoch vielfach zu einer starken Vignettierung, also einer Abschattung zum Bildrand hin. Ferner setzen solche Masken sehr enge Fertigungstoleranzen für das optische System voraus.

Ein optisches System, das diese Aufgabe für Endoskope mit Geradeausblick löst, ist in der unveröffentlichten deutschen Patenanmeldung DE 10 2016 214 025.6 offenbart worden.

WO 01/01186 A1 offenbart ein Ablenksystem für ein Endoskop mit einem Blickwinkel von bis zu 30°, das mindestens zwei prismatische Elemente als reflektierende Ablenkmittel umfasst, von denen das erste keilförmig ist. Die zur Abbildung kommenden Strahlen werden mehr als zweimal reflektiert, so dass Ablenkwinkel unterhalb von 30° ohne Verschlechterung der Bildqualität realisiert werden.

US 4,655,557 A offenbart ein optisches System für ein seitwärtsblickendes Endoskop mit einer rotierbaren Prismengruppe aus zwei Prismen, deren erstes Prisma ebenfalls keilförmig ist. Es findet eine doppelte Reflexion statt.

DE 24 58 306 A1 bezieht sich auf ein optisches System mit schräg nach vorn gerichtetem Sichtfeld zur Anwendung unter anderem in Endoskopen, bei denen die Beobachtungsrichtung unter einem vorgegebenen Winkel zur Längsrichtung des Endoskops liegt. Die distale optische Gruppe umfasst ein Sichtrichtungsänderungsprisma, ein Objektiv und ein optisches Bildübertragungselement, denen ein Korrekturprisma vorgeschaltet sein kann.

Es ist eine Aufgabe der Erfindung, ein verbessertes optisches System sowie ein verbessertes chirurgisches Instrument mit seitlicher Blickrichtung mit einem solchen optischen System bereitzustellen, wobei das optisches System unempfindlicher gegenüber von außerhalb eines Bildfeldes einfallenden Lichtbündeln sein soll.

Diese Aufgabe wird gelöst durch ein optisches System für ein seitwärts blickendes Endoskop, mit einem zentralen Strahlengang, der distal einen seitlichen Blickwinkel $\theta$ in Bezug auf eine Längsachse eines Endoskopschafts des Endoskops aufweist, umfassend eine distale optische Baugruppe mit einer Prismengruppe, die zur Umlenkung von aus einem um den seitlichen Blickwinkel herum definierten Sichtfeld einfallenden Licht in eine Richtung der Längsachse des Endoskopschafts mittels Reflexion an einer ersten Reflexionsfläche und einer zweiten Reflexionsfläche ausgebildet ist und drei Prismen umfasst, deren jeweils aneinander angrenzende Grenzflächen paarweise zueinander parallel angeordnet und durch jeweils einen Spalt getrennt sind, wobei an einer Grenzfläche zwischen einem Prisma und dem nachfolgenden Spalt Totalreflexion für einen Teil von außerhalb eines Sichtfelds einfallenden Lichts stattfindet, wobei die Prismengruppe eine zylindrische Einhüllende mit einem Durchmesser D aufweist, der entsprechend dem zur Verfügung stehenden Bauraum vorgegeben ist, und ein eingangsseitiges erstes Prisma der Prismengruppe keilförmig mit Keilwinkel $\beta$ und einer Wegstrecke a des zentralen Strahlengangs durch das erste Prisma ausgebildet ist, wobei eine Eintrittsfläche des ersten Prismas eine Länge L aufweist, definiert als eine Länge einer Schnittlinie der Eintrittsfläche mit der Ebene, die vom zentralen Strahlengang aufgespannt wird, das dadurch weitergebildet ist, dass das erste Prisma die Bedingungen

$$a < cos\theta \cdot tan\,\beta \cdot D/2 \qquad \text{sowie} \qquad L < D\,/\,cos\theta$$

erfüllt, wobei die Prismengruppe drei Prismen aufweist, wobei das zweite Prisma keilförmig, mit einem Keilwinkel $2\cdot\beta$, ausgebildet ist, wobei der Keilwinkel des zweiten Prismas an eine dem Keilwinkel des ersten Prismas gegenüberliegende Seite des ersten Prismas angrenzt und der Keilwinkel des ersten Prismas an eine dem Keilwinkel des zweiten Prismas gegenüberliegende Seite des zweiten Prismas angrenzt, so dass die Spalte zwischen den drei Prismen gegenüber dem zentralen Strahlengang in unterschiedliche Richtungen geneigt sind.

Die Prismengruppe stellt somit einen winkelabhängigen optischen Filter bereit, mit dem von außerhalb des Sichtfeldes in das optische System einfallende Lichtstrahlen aus dem Strahlengang herausreflektiert werden. Es tritt trotz des Seitwärtsblicks keine Vignettierung auf und es sind auch keine besonders hohen Anforderungen an die Zentrierung oder Justage der Prismengruppe im optischen System zu stellen. Eine solche Prismengruppe ist außerdem preisgünstig herzustellen und kann ohne großen konstruktiven Aufwand in das optische System integriert werden.

Es ist insbesondere vorgesehen, dass die Prismengruppe im optischen System drehbar aufgenommen ist. Beispielsweise ist die Prismengruppe innerhalb des optischen Systems um eine Achse drehbar, welche zumindest näherungsweise der optischen Achse des optischen Systems, insbesondere einer optischen Achse der distalen optischen Baugruppe des optischen Systems, entspricht. Durch eine Drehung der Prismengruppe ist es vorteilhaft möglich, das Sichtfeld variabel an verschiedenen Seiten mithilfe der Prismengruppe zu begrenzen.

Die beiden Bedingungen, die das erste Prisma erfüllt, sorgen dafür, dass die winkelabhängige optische Filterung mittels Totalreflexion vereint wird mit einem kompakten Aufbau, der in eine zylindrische Einhüllende passt, und der Seitwärtsblickwinkel $\theta$ berücksichtigt wird. Die Bedingungen sorgen dafür, dass der erste Keil bzw. das erste Prisma sehr dünn geschnitten ist. Gleichzeitig sind seine linke und rechte Seiten zylinderförmig und identisch mit den entsprechenden Seiten des zweiten und gegebenenfalls dritten Prismas. Damit können die Prismen leicht zusammengeklebt werden. Die Wegstrecke im ersten Prisma wird kurz gehalten, was insbesondere das Prismendesign für Prismen mit größerem Durchmesser vereinfacht.

Erfindungsgemäß weist die Prismengruppe drei Prismen auf, wobei das zweite Prisma keilförmig, insbesondere mit einem Keilwinkel $2\cdot\beta$, ausgebildet ist, wobei der Keilwinkel des zweiten Prismas an eine dem Keilwinkel des ersten Prismas gegenüberliegende Seite des ersten Prismas angrenzt und der Keilwinkel des ersten Prismas an eine dem Keilwinkel des zweiten Prismas gegenüberliegende Seite des zweiten Prismas angrenzt, so dass die Spalte zwischen den drei Prismen gegenüber dem zentralen Strahlengang in unterschiedliche Richtungen geneigt sind. Der zweite Spalt ist somit gegenüber dem ersten Spalt gedreht um einen Betrag, der dafür sorgt, dass Lichtstrahlen von außerhalb des Sichtbereichs symmetrisch total reflektiert aus dem Strahlengang entfernt werden, um Geisterbilder und Flares symmetrisch zu entfernen. Aus diesem Grund ist der Keilwinkel des mittleren Prismas doppelt so groß wie der Keilwinkel des ersten Prismas. Diese Auswahl erzielt eine Symmetrie der beiden Spalte gegenüber dem zentralen Strahlengang der Prismengruppe.

Vorzugsweise ist der Spalt oder sind die Spalte mit einem Medium gefüllt, das eine geringere optische Dichte hat als ein für die Prismen verwendetes Glas, wobei das Medium insbesondere ein Vakuum, eine inerte Atmosphäre oder Luft ist. Diese Auswahl sorgt dafür, dass eine Totalreflexion möglich wird, die beim Übergang von einem optisch dichteren Medium in ein optisch dünneres Medium auftritt, nicht aber beim Übergang von einem optisch dünneren in ein optisch dichteres Medium.

Vorteilhafterweise weist oder weisen das erste Prisma und/oder das zweite Prisma einen unteren Schnitt und/oder einen oberen Schnitt auf. Mit dieser Maßnahme wird es möglich, die oberen und/oder unteren Schnitte der Prismen bei der Montage als Anschlag zu verwenden. Die entsprechenden Schnitte bilden Abweichungen von der zylindrischen Einhüllenden, vorzugsweise Sehnen innerhalb des von der zylindrischen Einhüllenden gebildeten kreisförmigen Querschnitts. So können Plätze in einer Halterung gespart werden, damit mehr Elemente, beispielsweise Heizungselemente, im System eingebaut werden können. Vorzugsweise sind die unteren oder oberen Schnitte der Prismen in der Prismengruppe in ihrer Höhe gestaffelt ausgebildet. Dies erleichtert die Nutzung der Schnitte als Anschläge für die Montage weiter.

In einer vorteilhaften Weiterbildung sind eine proximale optische Baugruppe und wenigstens ein Bildsensor oder ein Okular proximal der proximalen optischen Baugruppe umfasst. Als proximale optische Baugruppe kann eine Linsengruppe verwendet werden, die das Licht aus der distalen optischen Baugruppe auf einen nachfolgenden Bildsensor oder, im Fall der Stereoendoskopie, auf ein paar von nachfolgenden Bildsensoren fokussiert. Die proximale Baugruppe kann auch eine Folge von Umlenkeinheiten sein, die das Licht zu einem Okular am proximalen Ende des Endoskops leiten.

Die distale optische Baugruppe umfasst vorteilhafterweise eine Eintrittslinse, die in Lichteinfallsrichtung vor der Eintrittsfläche des ersten Prismas angeordnet ist. Besonders bevorzugt ist es, wenn die Eintrittslinse auf der Eingangsfläche des ersten, eingangsseitigen, Prismas angeordnet ist. Hierdurch ergibt sich eine kompakte optische Anordnung.

Die der Erfindung zugrunde liegende Aufgabe wird auch durch ein seitwärts blickendes Endoskop mit einem zuvor beschriebenen erfindungsgemäßen optischen System gelöst.

Weitere Merkmale der Erfindung werden aus der Beschreibung erfindungsgemäßer Ausführungsformen zusammen mit den Ansprüchen und den beigefügten Zeichnungen ersichtlich. Erfindungsgemäße Ausführungsformen können einzelne Merkmale oder eine Kombination mehrerer Merkmale erfüllen.

Die Erfindung wird nachstehend ohne Beschränkung des allgemeinen Erfindungsgedankens anhand von Ausführungsbeispielen unter Bezugnahme auf die Zeichnungen beschrieben, wobei bezüglich aller im Text nicht näher erläuterten erfindungsgemäßen Einzelheiten ausdrücklich auf die Zeichnungen verwiesen wird. Es zeigen:

Fig. 1        ein optisches System in einem schematisch vereinfachten Längsschnitt gemäß dem Stand der Technik,
Fig. 2        ein optisches System in einem schematisch vereinfachten Längsschnitt,
Fig. 3        ein optisches System eines Stereo-Videoendoskops,
Fig. 4a), b)  eine Prismengruppe mit zwei Prismen nach dem Stand der Technik sowie Details des ersten Prismas der Prismengruppe,
Fig. 5        ein Beispiel einer Prismengruppe mit zwei Prismen, welches nicht unter den Schutzumfang fällt,
Fig. 6        ein Ausführungsbeispiel einer Prismengruppe mit drei Prismen,
Fig. 7        eine perspektivische Ansicht des ersten Prismas der Prismengruppe aus Fig. 6 und
Fig. 8a), b)  Details eines Ausführungsbeispiels einer Prismengruppe mit drei Prismen.

In den Zeichnungen sind jeweils gleiche oder gleichartige Elemente und/oder Teile mit denselben Bezugsziffern versehen, so dass von einer erneuten Vorstellung jeweils abgesehen wird.

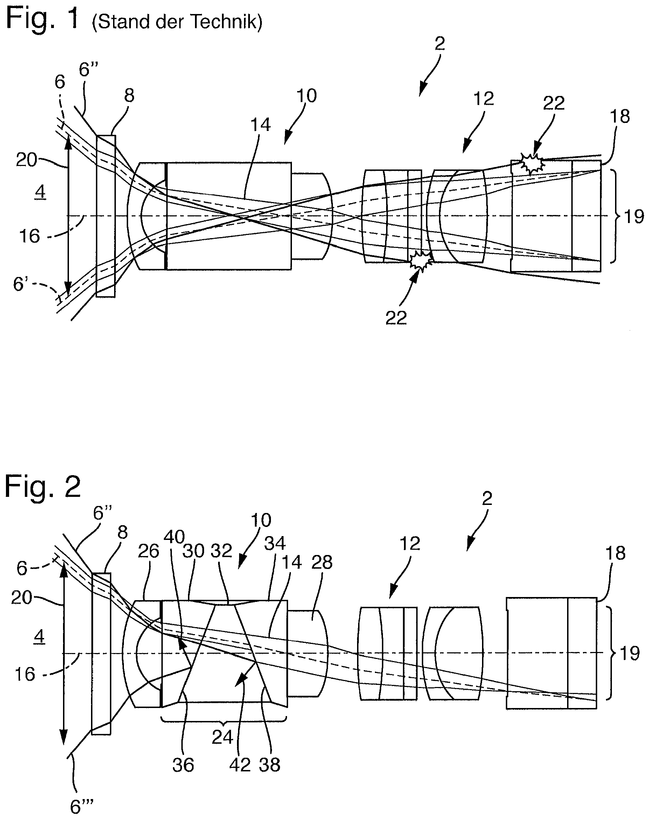

Fig. 1 zeigt ein optisches System 2 gemäß dem Stand der Technik in einer schematisch vereinfachten Längsschnittdarstellung. Aus einem Objektraum 4 einfallende Lichtbündel 6 (von denen aus Gründen der Übersichtlichkeit lediglich eines mit Bezugszeichen versehen ist) treffen zunächst auf ein Eintrittsfenster 8. Das optische System 2 ist beispielsweise ein Bauteil eines chirurgischen Instruments, ferner beispielsweise das optische System 2 eines Endoskops. Bei einem Endoskop schließt das Eintrittsfenster 8 den Innenraum eines Endoskopschafts an seinem distalen Ende gegenüber einem Außenraum bzw. Objektraum 4 hermetisch ab. Haben die Lichtbündel 6 das Eintrittsfenster 8 durchquert, treffen sie auf eine distale optische Baugruppe 10 und gelangen anschließend in eine proximale optische Baugruppe 12. Die distale und die proximale optische Baugruppe 10, 12 definieren einen Strahlengang 14 innerhalb des optischen Systems 2.

Im Objektraum 4 ist ein Sichtfeld 20 gelegen, welches durch einen horizontalen und einen vertikalen Bildwinkel definiert ist. Der in Fig. 1 gezeigte Längsschnitt zeigt beispielhaft einen Schnitt entlang einer vertikalen Ebene. Folglich ist der vertikale Bildwinkel zu sehen. Es handelt sich um den Winkel zwischen einer optischen Achse 16 des optischen Systems 2 und dem Lichtbündel 6, welches gerade noch auf eine lichtempfindliche Fläche 19 eines Bildsensors 18 trifft. Das Sichtfeld 20 ist in Fig. 1 schematisch mit einem Pfeil angedeutet. Die distale optische Baugruppe 10 und die proximale optische Baugruppe 12 bilden aus dem Sichtfeld 20 einfallende Lichtbündel 6, 6' auf die lichtempfindliche Fläche 19 des Bildsensors 18 ab.

Fallen jedoch Lichtbündel 6" von außerhalb des Sichtfeldes 20 in das optische System 2 ein, so verursachen diese diffuse Streuung und Reflexionen innerhalb des optischen Systems 2. Beispielsweise kommt es zu diffuser Streuung an einer Innenwand eines Tubus oder eines Linsenrohrs des optischen Systems 2. Dies ist in Fig. 1 mit sternförmigen Markierungen angedeutet, welche Streuzentren 22 andeuten sollen. Diese Reflexionen oder Streuungen verursachen "Flare" oder "Lense Flare", ein Phänomen, welches die Bildqualität des optischen Systems 2 nachteilig beeinflusst.

Fig. 2 zeigt ein optisches System 2 mit Geradeausblick gemäß der deutschen Patentanmeldung DE 10 2016 214 025.6 der Anmelderin, ebenfalls in einer vereinfachten und schematischen Längsschnittansicht entlang einer vertikalen Schnittebene. Das optische System 2 umfasst eine distale optische Baugruppe 10 sowie eine proximale optische Baugruppe 12. Die distale optische Baugruppe 10 und die proximale optische Baugruppe 12 definieren innerhalb des optischen Systems 2 einen Strahlengang 14. Aus dem Objektraum 4 von innerhalb des Sichtfeldes 20 in das optische System 2 einfallende Lichtbündel 6 (von denen beispielhaft lediglich eines dargestellt ist) werden auf eine lichtempfindliche Fläche 19 des Bildsensors 18 abgebildet.

Das optische System 2 gemäß dem dargestellten Ausführungsbeispiel umfasst eine im Strahlengang 14 angeordnete Prismengruppe 24. Die Prismengruppe 24 umfasst zumindest ein Prisma 30, 32, 34 und begrenzt das Sichtfeld 20 des optischen Systems 2 an zumindest einer Seite. Neben der Prismengruppe 24 umfasst die distale optische Baugruppe 10 noch eine Eintrittslinse 26 und eine Austrittslinse 28. Das zumindest eine Prisma 30, 32, 34 der Prismengruppe 24 umfasst eine Grenzfläche 36, 38, an der von außerhalb des Sichtfeldes 20 in das optische System 2 einfallende Lichtstrahlen 6" unter Totalreflexion aus dem Strahlengang 14 herausreflektiert werden.

Die in Fig. 2 gezeigte Prismengruppe 24 umfasst beispielhaft ein erstes Prisma 30, ein zweites Prisma 32 und ein drittes Prisma 34. Das erste Prisma 30 stellt eine erste Grenzfläche 36 bereit, an der ein erstes Lichtbündel 40 (angedeutet durch einen Pfeil) aus dem Strahlengang 14 herausreflektiert wird. Das zweite Prisma 32 stellt eine zweite Grenzfläche 36 bereit, an der ein zweites Lichtbündel 42 (ebenfalls angedeutet durch einen Pfeil) in einer anderen Richtung aus dem Strahlengang 14 herausreflektiert wird.

Die aus dem Strahlengang 14 herausreflektierten Lichtbündel treten von außerhalb des Sichtfeldes 20 als Lichtbündel 6" und 6‴ in das optische System 2 ein. Das in Fig. 2 an der Unterseite des optischen Systems 2 von außerhalb des Sichtfeldes 20 einfallende Lichtbündel 6‴ wird als erstes Lichtbündel 40 an der ersten Grenzfläche 36 totalreflektiert und so aus dem Strahlengang 14 entfernt. Das von außerhalb des Sichtfeldes 20 an der oberen Seite in das optische System 2 einfallende Lichtbündel 6" wird als zweites Lichtbündel 42 an der zweiten Grenzfläche 38 totalreflektiert und auf diese Weise aus dem Strahlengang 14 herausreflektiert.

Die Prismengruppe 24 begrenzt das Sichtfeld 20 an zwei einander gegenüberliegenden Seiten, beispielhaft an einer unteren und an einer oberen horizontalen Kante des Sichtfeldes 20. An diesen Seiten des Sichtfeldes 20 in das optische System 2 einfallende Lichtstrahlen 6", 6‴, welche von außerhalb des Sichtfeldes 20 einfallen, werden aus dem Strahlengang 14 herausreflektiert. Durch eine Drehung der Prismengruppe 24 um die optische Achse 16 kann in gleicher Weise eine Begrenzung, beispielsweise an den vertikalen Kanten des Sichtfeldes 20, erfolgen als an der linken oder der rechten Seite des Sichtfeldes 20. Hierzu müsste die Prismengruppe 24 um 90° um die optische Achse 16 gedreht werden, außerdem müsste sie auf den erforderlichen horizontalen Blickwinkel (der möglicherweise größer ist als der vertikale Blickwinkel) angepasst werden. Eine solche Anpassung erfolgt beispielsweise durch geeignete Wahl der Neigung der Grenzflächen 36, 38 gegenüber der optischen Achse 16.

Es ist ebenso möglich, eine weitere, in Fig. 2 nicht dargestellte, Prismengruppe 24 hinzuzufügen. Bei einem solchen Ausführungsbeispiel wäre eine erste Prismengruppe 24 wie die in Fig. 2 dargestellte Prismengruppe 24 angeordnet, eine zweite Prismengruppe wäre in Lichteinfallsrichtung folgend um 90° um die optische Achse 16 gedreht angeordnet. So könnte eine Begrenzung des Sichtfeldes 20 sowohl an den horizontalen als auch an den vertikalen Grenzen des Sichtfeldes 20 erreicht werden.

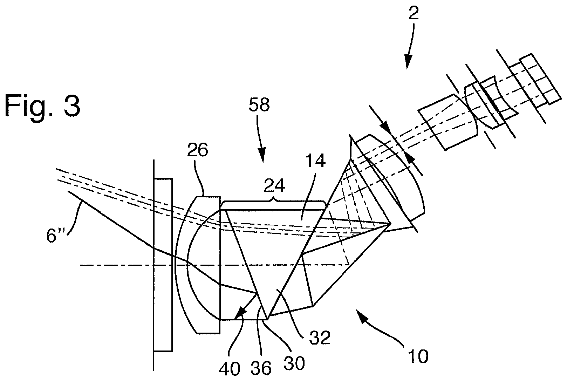

Fig. 3 zeigt ein weiteres optisches System 2 gemäß der deutschen Patentanmeldung DE 10 2016 214 025.6 der Anmelderin. Das optische System 2 ist beispielsweise das optische System 2 eines Stereo-Video-Endoskops mit Seitwärtsblick. Das optische System 2 umfasst als Teil der Umlenkprismengruppe 58 eine Prismengruppe 24, die eine Grenzfläche 36 umfasst, an der das von außerhalb des Sichtfeldes 20 einfallende Lichtbündel 6" als erstes Lichtbündel 40 aus dem Strahlengang 14 herausreflektiert wird. Zu diesem Zweck umfasst die Prismengruppe 24 das erste Prisma 30 und das zweite Prisma 32. Zwischen der ersten Grenzfläche 36 des ersten Prismas 30 ist erneut bevorzugt ein erster Luftspalt vorgesehen, so dass Totalreflexion an der Grenzfläche 36 stattfindet. Das erste und das zweite Prisma 30, 32 sind insbesondere so ausgestaltet, dass diese das erste Umlenkprisma 62 der Umlenkprismengruppe 58 ersetzen, also eine äquivalente optische Wirkung (abgesehen von der Totalreflexion von nicht aus dem Sichtfeld 20 kommenden Lichtbündeln 6") entfalten. Die Prismengruppe 24, welche jeweils ein Teil der distalen optischen Baugruppe 10 ist, entfernt unerwünschtes Streulicht direkt am Anfang des optischen Systems 2. Dies steigert die Abbildungsqualität des optischen Systems 2.

Die Prismengruppe aus Fig. 3 ist baulich aufwändig und groß bauend, da sie nicht in eine zylindrische Einhüllende eingepasst ist.

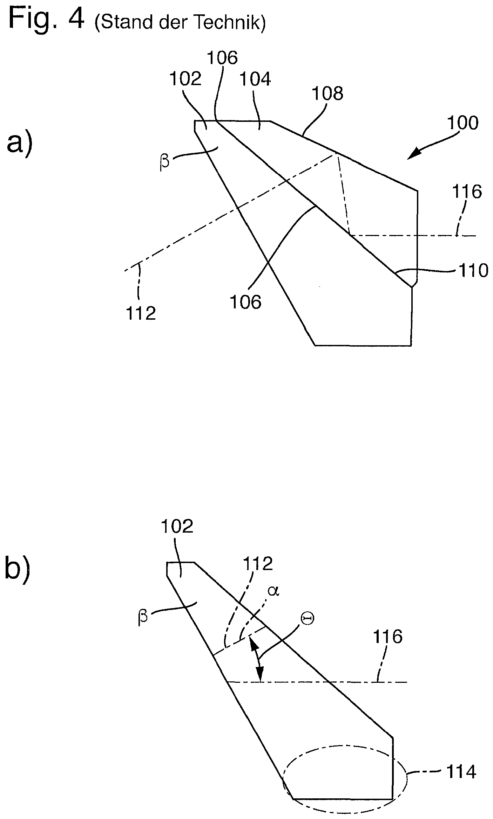

In Figur 4 ist eine distale optische Baugruppe 100 mit einer Prismengruppe mit zwei Prismen schematisch dargestellt, die aus dem Stand der Technik bekannt ist. Die Gruppe aus erstem Prisma 102 und zweitem Prisma 104 ist in Figur 4a) im Querschnitt dargestellt, zusammen mit dem zentralen Strahlengang 112. Zwischen den beiden Prismen 102 und 104 befindet sich ein Spalt 106, der mit Luft oder einem anderen Medium gefüllt ist.

Der zentrale Strahlengang 112 beginnt im Sichtfeld auf der linken Bildseite und verläuft zunächst unter einem Winkel zur zentralen Achse des Endoskops, die in der vorliegenden Darstellung horizontal verläuft. Der Strahlengang 112 tritt durch eine erste Grenzfläche in das erste Prisma 102 ein, tritt durch den Spalt 106 in das zweite Prisma 104 ein und wird an der verspiegelten hinteren Grenzfläche des zweiten Prismas 104 reflektiert. Der Strahlengang 112 verläuft innerhalb des zweiten Prismas zurück zum Spalt 106, wo eine Reflexion, beispielsweise eine Totalreflexion, an der zweiten Reflexionsfläche 110 stattfindet, die den zentralen Strahlengang 112 so umlenkt, dass er beispielsweise mit der zentralen Längsachse 116 des Endoskops zusammenfällt.

In Figur 4b) ist das erste Prisma 102 der Prismengruppe aus Figur 4a) mehr im Detail gezeigt. Das Prisma ist an der oberen Seite abgeschnitten, ist keilförmig ausgebildet und hat einen Keilwinkel $\beta$. Der Teil des zentralen Strahlengangs 112, der durch das erste Prisma 102 verläuft, hat eine Länge a, und zur zentralen Längsachse 116 des Endoskops einen Winkel $\theta$, der somit den Seitwärtsblickwinkel des Endoskops definiert. Wie sowohl in Figur 4a) als auch in Figur 4b) zu erkennen ist, wird für den optischen Strahlengang um den zentralen Strahlengang 112 herum vor allem der obere Teil der Prismengruppe verwendet, während der untere Teil optisch nicht verwendet wird. Das optisch unbenutzte Glasvolumen ist mit dem Bezugszeichen 114 bezeichnet und stellt einen quasi toten Raum oder Ballast dar.

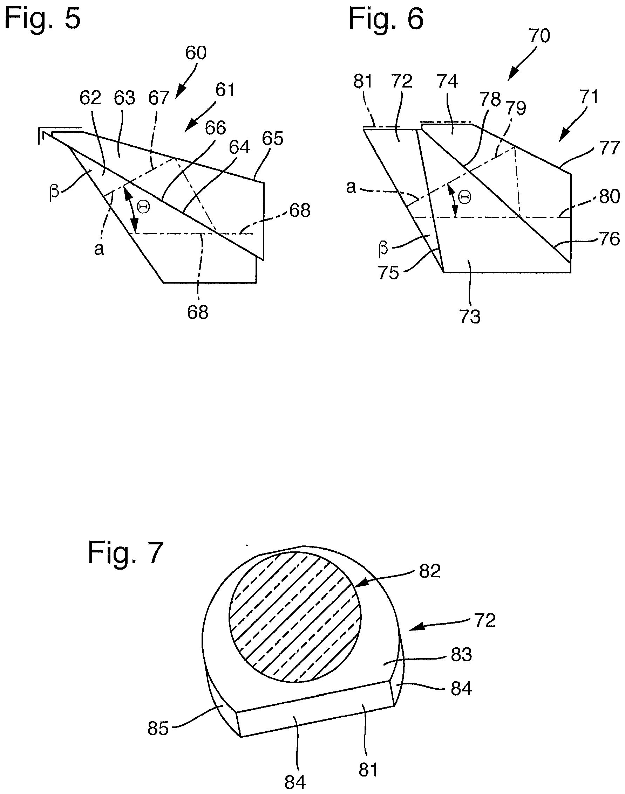

Figur 5 zeigt ein nicht-erfindungsgemäßes Beispiel einer Prismengruppe 61 eines optischen Systems 60. Hierbei handelt es sich um eine Prismengruppe 61 mit zwei Prismen, nämlich einem ersten Prisma 62 und einem zweiten Prisma 63, die durch einen Spalt 64 voneinander getrennt sind. Ebenfalls gezeigt sind in Figur 5 die Wegstrecke a des zentralen Strahlengangs 67 durch das erste Prisma 62, der Keilwinkel $\beta$ des ersten Prismas 62 sowie der seitliche Blickwinkel $\theta$. Der zentrale Strahlengang 67 verläuft ab dem Eintritt durch das erste Prisma 62, den Spalt 64, das zweite Prisma 63, erfährt eine Reflexion an der ersten Reflexionsfläche 65 sowie eine weitere Reflexion an der zweiten Reflexionsfläche 66, wo der zentrale Strahlengang 67 in die zentrale Längsachse 68 des Endoskops eintritt. Der Durchgang durch den Spalt 64 sorgt dafür, dass Lichtstrahlen, die unter einem größeren Winkel von weiter oben in Figur 5 eintreten, durch Totalreflexion weg reflektiert werden und aus dem Strahlengang entfallen.

Figur 6 zeigt ein Ausführungsbeispiel einer Prismengruppe 71 eines optischen Systems 70 gemäß der vorliegenden Erfindung. Diese Prismengruppe 71 hat drei Prismen 72, 73 und 74, die durch zwei Spalte 75, 76 voneinander getrennt sind. Ebenfalls erkennbar sind die Wegstrecke a des zentralen Strahlengangs 79 durch das erste Prisma 72, der Keilwinkel $\beta$ des ersten Prismas 72 sowie der Seitwärtsblickwinkel $\theta$ des optischen Systems 70, der in diesem Ausführungsbeispiel 30° beträgt. Ebenso wie in den vorherigen Beispielen wird der zentrale Strahlengang 79 an einer ersten Reflexionsfläche 77 und an einer zweiten Reflexionsfläche 78 aus der seitlichen Eintrittsrichtung in die symmetrieachsparallele Richtung des Endoskopschaft doppelt reflektiert. Es ist erkennbar, dass beispielsweise das erste Prisma 72 einen oberen Schnitt 81 aufweist. Auch die anderen Prismen 73, 74 sind an ihren Oberseiten bzw. Unterseiten beschnitten. Im Vergleich zu dem bekannten Prismensystem gemäß beispielsweise Figur 4 ist auffällig, dass nach unten hin sehr viel weniger totes Glasvolumen vorhanden ist.

Figur 6 zeigt bereits ansatzweise, dass die beiden Spalte 75, 76 gegenüber dem ersten Abschnitt des zentralen Strahlengangs 79 spiegelbildlich zueinander angeordnet sind, sodass sie eine symmetrische Abweisung von Lichtstrahlen durch Totalreflexion bewirken, die von weiter oben oder von weiter unten außerhalb des Sichtfelds in die Prismengruppe 71 eintreten.

Figur 7 zeigt eine perspektivische Darstellung des ersten Prismas 72 der Prismengruppe 71 aus Figur 6, in dem erkennbar ist, dass dieses Prisma 72 in eine zylindrische Einhüllende 85 eingepasst ist. Der obere Schnitt 81 des ersten Prismas 72 ist ebenso erkennbar wie ein transparenter Bereich 82, der für den gewünschten Strahlengang vorgesehen ist sowie ein intransparent beschichteter Rand 83, der insbesondere mit einer Anti-Flare-Beschichtung 84 beschichtet ist.

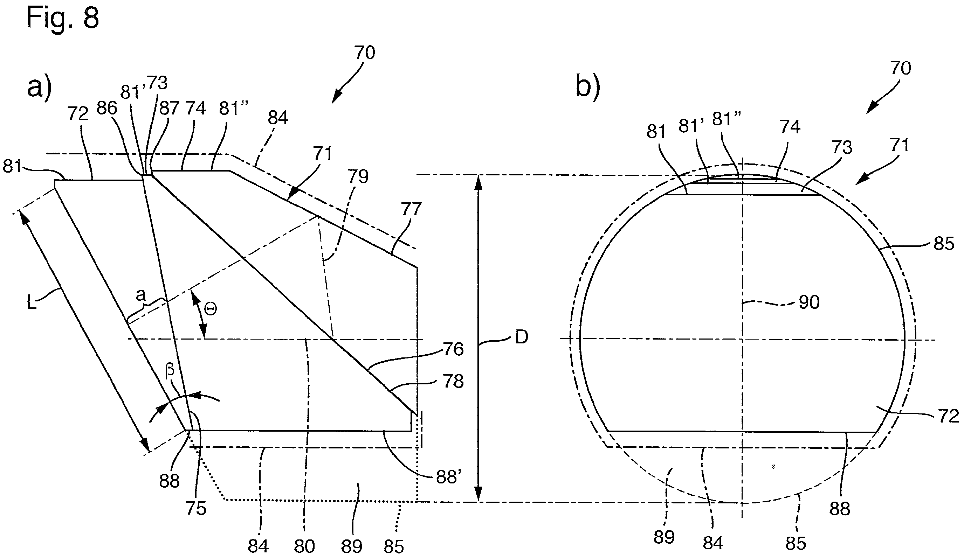

Figur 8a) zeigt eine Querschnittsdarstellung durch das Ausführungsbeispiel gemäß Figur 6 in größerem Detail. Die Ebene der Querschnittsdarstellung entspricht der Symmetrieebene 90 des optischen Systems 70, die in Figur 8b) dargestellt ist.

Neben den Winkeln $\beta$ und $\theta$, die wie zuvor definiert sind, und der Länge a des Abschnitts des zentralen Strahlengangs 79, der durch das erste Prisma 72 verläuft, sind in Fig. 8a) noch die Länge L der Eingangsfläche des ersten Prismas 72 sowie der Durchmesser D der Einhüllenden 85 der Prismengruppe 71 des optischen Systems 70 dargestellt. Bei der Länge L der Eintrittsfläche des ersten Prismas 72 handelt es sich um die Länge der Eintrittsfläche in der Symmetrieebene 90 der Prismengruppe, in der auch der vollständige zentrale Strahlengang 79 verläuft. Der Durchmesser D der Einhüllenden ist gleich dem Durchmesser der kreisförmigen Projektion der Einhüllenden, die in Figur 8b) gezeigt ist. Figur 8b) zeigt zusätzlich die Abfolge von oberen Schnitten 81, 81', 81" der 3 Prismen 72, 73 und 74, die eine Stufenfolge bildet, die auch in Figur 8a) erkennbar ist. Diese bilden Anschlagkanten 86, 87 an der oberen Seite der Prismengruppe 71. Ebenfalls in Figur 8 gezeigt ist die Anti-Flare-Beschichtung 84 der Prismengruppe 71 sowie die unteren Schnitte 88, 88', 88" der Prismen 72, 73 und 74.

In Figur 8a) ist ferner zu erkennen, dass nach der zweiten Spiegelung der zentrale Strahlengang 79 mit der zentralen Längsachse 80 des Endoskopschafts zusammenfällt. Ebenfalls erkennbar ist, dass im unteren Bereich das Material der Prismengruppe 71 die Einhüllende 85 nicht vollständig ausfüllt, sondern ein unterer Leerraum verbleibt. Dieser wird im voll montierten Zustand einerseits durch einen entsprechenden Halter eingenommen, der wiederum Platz haben kann für Lichtzuleitungsmittel, beispielsweise Lichtleitfasern.

Die Geometrie des ersten Prismas 73 erfüllt die Bedingungen

$$a < \cos\theta \cdot \tan\beta \cdot D/2$$

sowie

$$L < D / \cos\theta.$$

Mit diesen Bedingungen ist es zuverlässig möglich, kompakte Prismengruppen zu finden, die mit wenig optischem Totvolumen mit einem Seitwärtsblick und gleichzeitig Totalreflexion von seitlich eintretenden Lichtstrahlen an dem Spalt oder den Spalten der Prismengruppe in eine zylindrische Einhüllende einpassbar sind und somit wenig Bauraum benötigen.

So ist es beispielsweise möglich, bei vorgegebenem Seitwärtsblickwinkel $\theta$ den Winkel, ab dem von außerhalb des geplanten Sichtfelds eintretende Lichtstrahlen mittels Totalreflexion entfernt werden sollen, über den Keilwinkel $\beta$ des ersten Prismas 72 und gegebenenfalls den entsprechenden Keilwinkel des zweiten Prismas 73 einzustellen. Die Wegstrecke a lässt sich dann über eine Verschiebung des ersten Prismas 72 an der Grenzfläche zum zweiten Prisma 73 einstellen. Der Durchmesser D kann vorgegeben werden entsprechend dem zur Verfügung stehenden Bauraum, während die Länge L der Eintrittsfläche des ersten Prismas 72 sich anschließend aus der Kombination der gewählten Parameter und den noch zu wählenden Schnitten gegenüber der zylindrischen Einhüllenden ergibt.

Alle genannten Merkmale, auch die den Zeichnungen allein zu entnehmenden sowie auch einzelne Merkmale, die in Kombination mit anderen Merkmalen offenbart sind, werden allein und in Kombination als erfindungswesentlich angesehen. Erfindungsgemäße Ausführungsformen können durch einzelne Merkmale oder eine Kombination mehrerer Merkmale erfüllt sein. Die Erfindung ist in den anhängenden Ansprüchen definiert. Merkmale, die mit "insbesondere" oder "vorzugsweise" gekennzeichnet sind, als fakultative Merkmale zu verstehen.

Bezugszeichenliste

| | |
|------|-----------------------------------|
| 2 | optisches System |
| 4 | Objektraum |
| 6, 6', 6", 6‴ | Lichtbündel |
| 8 | Eintrittsfenster |
| 10 | distale optische Baugruppe |
| 12 | proximale optische Baugruppe |
| 14 | Strahlengang |
| 16 | optische Achse |
| 18 | Bildsensor |
| 19 | lichtempfindliche Fläche |
| 20 | Sichtfeld |
| 22 | Streuzentrum |
| 24 | Prismengruppe |
| 26 | Eintrittslinse |
| 28 | Austrittslinse |
| 30 | erstes Prisma |
| 32 | zweites Prisma |
| 34 | drittes Prisma |
| 36 | erste Grenzfläche |
| 38 | zweite Grenzfläche |
| 40 | erstes Lichtbündel |
| 42 | zweites Lichtbündel |
| 58 | Umlenkprismengruppe |
| 60 | optisches System |
| 61 | Prismengruppe |
| 62 | erstes Prisma |
| 63 | zweites Prisma |
| 64 | Spalt |
| 65 | erste Reflexionsfläche |
| 66 | zweite Reflexionsfläche |
| 67 | zentraler Strahlengang |
| 68 | Längsachse des Endoskopschafts |
| 70 | optisches System |
| 71 | Prismengruppe |
| 72 | erstes Prisma |
| 73 | zweites Prisma |
| 74 | drittes Prisma |
| 75 | erster Spalt |
| 76 | zweiter Spalt |
| 77 | erste Reflexionsfläche |
| 78 | zweite Reflexionsfläche |
| 79 | zentraler Strahlengang |
| 80 | Längsachse des Endoskopschafts |
| 81, 81',81" | oberer Schnitt |
| 82 | transparenter Bereich |
| 83 | intransparent beschichteter Rand |

| 84 | Anti-Flare-Beschichtung |
|----|----|
| 85 | zylindrische Einhüllende |
| 86, 87 | Anschlagkante |
| 88, 88', 88" | unterer Schnitt |
| 89 | unterer Leerraum |
| 90 | Symmetrieebene |
| 100 | distale optische Baugruppe |
| 102 | erstes Prisma |
| 104 | zweites Prisma |
| 106 | Spalt |
| 108 | erste Reflexionsfläche |
| 110 | zweite Reflexionsfläche |
| 112 | zentraler Strahlengang |
| 114 | optisch unbenutztes Glasvolumen |
| 116 | Längsachse des Endoskopschafts |

**Patentansprüche**

1. Optisches System (60, 70) für ein seitwärts blickendes Endoskop, mit einem zentralen Strahlengang (79), der distal einen seitlichen Blickwinkel $\theta$ in Bezug auf eine Längsachse (80) eines Endoskopschafts des Endoskops aufweist, umfassend eine distale optische Baugruppe mit einer Prismengruppe (71), die zur Umlenkung von aus einem um den seitlichen Blickwinkel herum definierten Sichtfeld einfallenden Licht in eine Richtung der Längsachse (80) des Endoskopschafts mittels Reflexion an einer ersten Reflexionsfläche (77) und einer zweiten Reflexionsfläche (78) ausgebildet ist und drei Prismen (72, 73, 74) umfasst, deren jeweils aneinander angrenzende Grenzflächen paarweise zueinander parallel angeordnet und durch jeweils einen Spalt (75, 76) getrennt sind, wobei an einer Grenzfläche zwischen einem Prisma (72, 73) und dem nachfolgenden Spalt (75, 76) Totalreflexion für einen Teil von außerhalb eines Sichtfelds einfallenden Lichts stattfindet, wobei die Prismengruppe (71) eine zylindrische Einhüllende (85) mit einem Durchmesser D aufweist, der entsprechend dem zur Verfügung stehenden Bauraum vorgegeben ist, und ein eingangsseitiges erstes Prisma (72) der Prismengruppe (71) keilförmig mit Keilwinkel $\beta$ und einer Wegstrecke a des zentralen Strahlengangs (79) durch das erste Prisma (72) ausgebildet ist, wobei eine Eintrittsfläche des ersten Prismas (72) eine Länge L aufweist, definiert als eine Länge einer Schnittlinie der Eintrittsfläche mit der Ebene, die vom zentralen Strahlengang (79) aufgespannt wird, **dadurch gekennzeichnet, dass** das erste Prisma (72) die Bedingungen

$$a < cos\theta \cdot tan\,\beta \cdot D/2 \qquad sowie \qquad L < D\,/\,cos\theta$$

erfüllt, wobei das zweite Prisma (73) keilförmig, mit einem Keilwinkel 2·$\beta$, ausgebildet ist, wobei der Keilwinkel des zweiten Prismas (73) an eine dem Keilwinkel des ersten Prismas (72) gegenüberliegende Seite des ersten Prismas angrenzt und der Keilwinkel des ersten Prismas an eine dem Keilwinkel des zweiten Prismas (73) gegenüberliegende Seite des zweiten Prismas (73) angrenzt, so dass die Spalte (75, 76) zwischen den drei Prismen (72, 73, 74) gegenüber dem zentralen Strahlengang (79) in unterschiedliche Richtungen geneigt sind.

2. Optisches System nach Anspruch 1, **dadurch gekennzeichnet, dass** die Spalte (75, 76) mit einem Medium gefüllt sind, das eine geringere optische Dichte hat als ein für die Prismen (72, 73, 74) verwendetes Glas.

3. Optisches System nach Anspruch 2, **dadurch gekennzeichnet, dass** das Medium ein Vakuum, eine inerte Atmosphäre oder Luft ist.

4. Optisches System nach einem der Ansprüche 1 bis 3, **dadurch gekennzeichnet, dass** das erste Prisma (72) einen unteren Schnitt (88) und/oder einen oberen Schnitt (81) aufweist.

5. Optisches System nach einem der Ansprüche 1 bis 4, **dadurch gekennzeichnet, dass** das zweite Prisma (73) einen unteren Schnitt (88') und/oder einen oberen Schnitt (81') aufweist.

6. Optisches System nach einem der Ansprüche 1 bis 5, **dadurch gekennzeichnet, dass** die unteren Schnitte (88, 88') oder oberen Schnitte (81, 81', 81") der Prismen (72, 73, 74) in der Prismengruppe (71) in ihrer Höhe gestaffelt ausgebildet sind.

7. Optisches System nach einem der Ansprüche 1 bis 6, **dadurch gekennzeichnet, dass** eine proximale optische Baugruppe (12) und wenigstens ein Bildsensor (18) oder ein Okular proximal der proximalen optischen Baugruppe (12) umfasst sind.

8. Optisches System nach einem der Ansprüche 1 bis 7, **dadurch gekennzeichnet, dass** die distale optische Baugruppe eine Eintrittslinse (26) umfasst, die in Lichteinfallsrichtung vor der Eintrittsfläche des ersten Prismas (72) angeordnet ist.

9. Seitwärts blickendes Endoskop mit einem optischen System nach einem der Ansprüche 1 bis 8.

**Claims**

1. An optical system (60, 70) for a side-viewing endoscope with a central beam path (79) that has a distal lateral viewing angle $\theta$ relative to a longitudinal axis (80) of an endoscope shaft of the endoscope, comprising a distal optical assembly having a prism group (71) that is designed to deflect incident light from a field of view defined around the lateral viewing angle into a direction of the longitudinal axis (80) of the endoscope shaft by means of reflection at a first reflective surface (77) and at a second reflective surface (78), and comprises three prisms (72, 73, 74), the mutually adjacent boundary surfaces of which are arranged in pairs parallel to each other and are separated by a gap (75, 76) in each case, wherein total internal reflection of some of the incident light from outside a field of view takes place at a boundary surface between a prism (72, 73) and the subsequent gap (75, 76), wherein the prism group (71) has a cylindrical envelope (85) with a diameter D that is predetermined according to the available installation space, and an input-side first prism (72) of the prism group (71) is designed wedge-shaped with wedge angle $\beta$ and an optical path length a of the central beam path (79), wherein an entry surface of the first prism (72) has a length L, defined as a length of a line of intersection of the entry surface with the plane that is spanned by the central beam path (79), **characterized in that** the first prism (72) meets the conditions:

$$a < cos\theta \cdot tan\,\beta \cdot D/2 \qquad and \qquad L < D\,/\,cos\theta,$$

wherein the second prism (73) is designed wedge-shaped, with a wedge angle of $2\cdot\beta$, wherein the wedge angle of the second prism (73) borders a side of the first prism opposite the wedge angle of the first prism (72), and the wedge angle of the first prism borders a side of the second prism (73) opposite the wedge angle of the second prism (73) so that the gaps (75, 76) between the three prisms (72, 73, 74) are angled relative to the central beam path (79) in different directions.

2. The optical system according to claim 1, **characterized in that** the gaps (75, 76) are filled with a medium that has a lesser optical density than a glass used for the prisms (72, 73, 74).

3. The optical system according to claim 2, **characterized in that** the medium is a vacuum, an inert atmosphere or air.

4. The optical system according to one of claims 1 to 3, **characterized in that** the first prism (72) has a lower cut (88) and/or an upper cut (81).

5. The optical system according to one of claims 1 to 4, **characterized in that** the second prism (73) has a lower cut (88') and/or an upper cut (81').

6. The optical system according to one of claims 1 to 5, **characterized in that** the lower cuts (88, 88') or upper cuts (81, 81', 81") of the prisms (72, 73, 74) in the prism group (71) are formed stacked in height.

7. The optical system according to one of claims 1 to 6, **characterized in that** a proximal optical assembly (12) and at least one image sensor (18) or an eyepiece are comprised proximal to the proximal optical assembly (12).

8. The optical system according to one of claims 1 to 7, **characterized in that** the distal optical assembly comprises an entry lens (26) that is arranged in the direction of incident light before the entry surface of the first prism (72).

9. A side-viewing endoscope with an optical system according to one of claims 1 to 8.


**Revendications**

1. Système optique (60, 70) pour un endoscope à vision latérale, avec un trajet de faisceau central (79) qui présente distalement un angle de vision latérale $\theta$ par rapport à un axe longitudinal (80) d'une tige d'endoscope de l'endoscope, comprenant un ensemble optique distal avec un groupe de prismes (71), qui est conçu pour dévier la lumière incidente d'un champ de vision défini autour de l'angle de vision latérale dans une direction de l'axe longitudinal (80) de la tige de l'endoscope au moyen d'une réflexion sur une première surface de réflexion (77) et une deuxième surface de réflexion (78) et qui comprend trois prismes (72, 73, 74) dont les surfaces limites mutuellement contigües sont agencées par paires parallèlement les unes aux autres et sont mutuellement séparées par une fente (75, 76), une réflexion totale se produisant au niveau d'une surface limite entre un prisme (72, 73) et la fente suivante (75, 76), pour une partie de la lumière incidente en dehors d'un champ de vision, le groupe de prismes (71) présentant une enveloppe cylindrique (85) d'un diamètre D qui est prédéfini en fonction de l'espace de construction disponible, et un premier prisme (72) côté entrée du groupe de prismes (71) est formé en forme de coin avec un angle au sommet $\beta$ et un trajet a du trajet de faisceau central (79) à travers le premier prisme (72), une face d'entrée du premier prisme (72) ayant une longueur L définie comme une longueur d'une ligne d'intersection de la face d'entrée avec le plan défini par le trajet de faisceau central (79), **caractérisé en ce que** le premier prisme (72) satisfait aux conditions

$$a < cos\theta \cdot tan\ \beta \cdot D/2 \quad \text{ainsi que} \quad L < D\ /\ cos\theta$$

le deuxième prisme (73) étant en forme de coin, avec un angle au sommet $2 \cdot \beta$, l'angle au sommet du deuxième prisme (73) étant adjacent à un côté du premier prisme opposé à l'angle au sommet du premier prisme (72) et l'angle au sommet du premier prisme étant adjacent à un côté du deuxième prisme (73) opposé à l'angle au sommet du deuxième prisme (73), de sorte que les fentes (75, 76) entre les trois prismes (72, 73, 74) sont inclinées dans des directions différentes par rapport au trajet optique central (79).

2. Système optique selon la revendication 1, **caractérisé en ce que** les fentes (75, 76) sont remplies d'un milieu ayant une densité optique inférieure à celle d'un verre utilisé pour les prismes (72, 73, 74).

3. Système optique selon la revendication 2, **caractérisé en ce que** le milieu est un vide, une atmosphère inerte ou de l'air.

4. Système optique selon l'une des revendications 1 à 3, **caractérisé en ce que** le premier prisme (72) présente une section inférieure (88) et/ou une section supérieure (81).

5. Système optique selon l'une des revendications 1 à 4, **caractérisé en ce que** le deuxième prisme (73) présente une section inférieure (88') et/ou une section supérieure (81').

6. Système optique selon l'une des revendications 1 à 5, **caractérisé en ce que** les sections inférieures (88, 88') ou les sections supérieures (81, 81', 81") des prismes (72, 73, 74) sont échelonnées en hauteur dans le groupe de prismes (71).

7. Système optique selon l'une quelconque des revendications 1 à 6, **caractérisé en ce qu'**un ensemble optique proximal (12) et au moins un capteur d'image (18) ou un oculaire sont inclus à l'ensemble optique proximal (12), de façon proximale.

8. Système optique selon l'une des revendications 1 à 7, **caractérisé en ce que** l'ensemble optique distal comprend une lentille d'entrée (26) disposée en amont de la face d'entrée du premier prisme (72) dans la direction d'incidence de la lumière.

9. Endoscope à vision latérale comprenant un système optique selon l'une quelconque des revendications 1 à 8.

# Fig. 1 (Stand der Technik)

# Fig. 2

Fig. 3

# Fig. 4 (Stand der Technik)

a)

b)

# Fig. 5

# Fig. 6

# Fig. 7

Fig. 8

EP 3 555 686 B1

**IN DER BESCHREIBUNG AUFGEFÜHRTE DOKUMENTE**

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- DE 102016214025
- WO 0101186 A1
- US 4655557 A
- DE 2458306 A1